# EUROPEAN PATENT SPECIFICATION

(11) **EP 4 144 293 B1**
(45) Date of publication and mention of the grant of the patent: **01.01.2025**
(21) Application number: 22192519.1
(22) Date of filing: 29.08.2022
(51) Int. Cl.: A61B 5/157, A61M 1/02, A61B 5/15

(54) **BLOOD PACK DONATION KIT FOR BIOMARKER COLLECTION DURING WHOLE BLOOD DONATION**
BLUTPACKUNGSSPENDENKIT ZUR SAMMLUNG VON BIOMARKERN WÄHREND DER VOLLBLUTSPENDUNG
KIT DE DON DE POCHE DE SANG POUR LA COLLECTE DE BIOMARQUEURS LORS D'UN DON DE SANG TOTAL

(30) Priority: 07.09.2021 US 202163241390 P
(43) Date of publication of application: 08.03.2023
(73) Proprietor: Fenwal, Inc., Lake Zurich, IL 60047 (US)
(72) Inventor: MADSEN, James, Lake Zurich, 60047 (US)
(74) Representative: Maikowski & Ninnemann Patentanwälte Partnerschaft mbB

(56) References cited:
- WO-A1-2010/103051
- WO-A2-02/05059
- US-A1- 2005 148 993
- US-A1- 2005 209 518

## Description

### BACKGROUND

### Field of the Disclosure

The invention relates to a blood collection kit. More particularly, the invention relates to devices used during whole blood donation and collection of biomarkers.

### Description of Related Art

Whole blood (hereinafter "WB") donation procedures typically are conducted with the sole purposed of WB collection. However, there is a rise in development of new in vitro diagnostics (hereinafter "IVD"), which are tests done on samples, such as blood or tissue, taken from a human subject. IVD can detect diseases or other conditions and may be used to monitor overall health and to potentially to help cure, treat or prevent diseases. IVD also can be used in precision medicine to identify patients who are likely to benefit from specific treatments or therapies.

Lab-on-a-chip (hereinafter "LOC") devices are commonly used in IVD. Such devices integrate one or several laboratory functions on a single integrated circuit (or chip) to achieve automation and high-throughput screening. LOC devices can handle extremely small fluid volumes, down to less than pico-liters.

LOC technology may soon become an important part of efforts to improve global health, particularly through the development of point-of-care (hereinafter "POC") testing devices. In countries with few healthcare resources, infectious diseases that would be treatable in a developed nation are often deadly. In some cases, poor healthcare clinics have the drugs to treat a certain illness, but lack the diagnostic tools to identify patients who should receive the drugs. Many researchers believe that LOC technology may be a key to powerful new diagnostic instruments. The goal of these researchers is to create microfluidic chips that will allow healthcare providers to perform diagnostic tests such as immunoassays and nucleic acid assays with no laboratory support. Development of LOC devices may provide numerous advantages, which are specific to their application.

Within the field of IVD, small volumes of WB may be collected from patients for analysis of circulating biomarkers. The analysis of plasma biomarkers can diagnose many diseases, such as cancer, Alzheimer's disease, or sepsis. Typically, plasma is separated from WB before analysis to prevent contamination of the biomarkers by the presence of leukocytes, erythrocytes and hemolysis, which could increase test variability and reduce test accuracy.

The current state of the art for acquiring plasma for LOC devices consists of either using a traditional bench-top centrifuge or using plasma separation filters as for example described in WO 02/05059 A2. In turn, each company developing a LOC device also develops its own in-vitro diagnostic device to collect biomarkers on a disposable cartridge. These separate processes and devices unfortunately do not lend themselves to point-of-care devices for wide-spread use or use during routine WB donations.

### SUMMARY

Insofar as the term embodiment or aspect or alternative is used in the following, or features are presented as being optional, this should be interpreted in such a way that the only protection sought is that of the invention claimed and defined in the appended claims.

The prospect of direct biomarker collection during WB donation could provide significant advantages in costs, efficiencies and timeliness. This may be facilitated by providing for use of LOC devices that provide for separation of biomarkers during WB donation, which will be the context for use of "LOC device(s)" hereinafter.

This disclosure seeks to provide a standardized blood pack donation system configured for use with a LOC device for biomarker separation and collection during WB donation. Many biotechnology companies are working to develop such LOC devices to separate, collect and/or detect biomarkers for diseases. These LOC devices typically require low flow rates as biomarkers are separated from WB. The invention of the present disclosure would provide a standardized blood pack donation system or kit, which would allow for LOC device testing during a routine blood donation procedure, such as for example a 500ml WB donation. Thus, a blood pack device and methods of using the same of the present disclosure permit WB donation and facilitate simultaneous use of a LOC device for separation and collection of biomarkers for further analysis.

It will be appreciated that the blood pack donation systems disclosed herein are configured for use with any of a variety of LOC devices, which may be provided by various manufacturers for use in separation, collection and screening for any one or more of a number of different biomarkers. Thus, makers of LOC devices may integrate their respective devices for use in testing in blood donation centers, clinics, medical facilities or other locations where routine WB donation may be conducted. As such, WB donors would have an option of being screened for disease marking biomarkers during a routine WB donation procedure, such as for example a 500ml WB donation. This would provide much broader access to biomarker screening and early disease detection for routine WB donors.

The availability of such an advantageous standardized blood pack donation system or kit for use with LOC devices in WB donation centers or other facilities would readily and conveniently enable screening many donors for disease biomarkers, while reducing costs and the logistics involved in developing and stocking individual systems from each manufacturer. It will be appreciated that this would be important to early diagnosis, prevention and treatment of cancer or any number of different diseases. A standardized system or kit may be coupled with a variety of LOC devices to yield many applications for any number of biomarkers. The convenience could prompt offers by blood donation centers, clinics or medical facilities to donate WB and screen for particular biomarkers, including but not limited to genetic material, cell free DNA, exosomes, or any particular bloodborne biomarker for a disease.

The system or kit may take advantage of microfluidics in line to the WB collection container. For example, flow may be driven via the donor's blood pressure. The WB collection container also may be relocated to hang at a greater height to reverse flow and return WB to the donor, if desired, such as during a multiple pass collection procedure. The potential to collect any biomarker by using a microfluidic or LOC device is quite broad. Thus, in a single pass collection procedure, depending on the configuration of the system, at least some of the WB that is collected during donation is run through a LOC device for separation, before reaching a WB collection container.

The WB collection container may have any configuration and selected volume and, when combined into the system, must have at least one opening. Thus, the opening may be preexisting and connected to a flow path, or may be formed when the WB collection container is being connected to a flow path within the system. In turn, the biomarkers separated out by the LOC device are collected and transferred to a biomarker collection container. The biomarker collection container also may have any configuration, such as a bag, tube, syringe or other suitable structure, and any selected volume, but generally will be much smaller than the WB collection container. When combined into the system, the biomarker collection container also must have at least one opening. Thus, the opening may be preexisting and connected to a flow path, or may be formed when the biomarker collection container is being connected to a flow path within the system.

An alternative second example system may be provided to utilize a single pass collection procedure or a multiple pass collection procedure. The second example system includes a bypass line or flow path between the donor and WB collection container. This permits procedures in which some, none or all of the WB bypasses the LOC device. The second example system may be employed in a multiple pass collection procedure, such as may include three steps. In the first step (step one), flow through the LOC device is permitted and flow through the bypass line or flow path may range from being fully or partially permitted to being prevented, depending on the portion of the donated WB intended to pass through the LOC device. Accordingly, at least some of the WB that is collected during donation from a donor is run through a LOC device for separation, before reaching a WB collection container. In turn, the biomarkers separated out by the LOC device are collected and transferred via a separate flow path to a biomarker collection container.

In a second step (step two) of the multiple pass collection procedure, flow is reversed from the WB collection container and all of the flow is permitted through the bypass line or flow path, while being entirely prevented from flowing back through the LOC device and thereby to the biomarker collection container. In a third step (step three), at least some of the WB that is collected during donation from a donor again is run through a LOC device for separation, before reaching the WB collection container. As with the first step, flow through the bypass line or flow path may range from being fully or partially permitted to being prevented. WB is collected again and at least some of the WB that is collected is run through the LOC device before reaching the WB collection container. The biomarkers separated out by the LOC device are collected and transferred to the biomarker collection container. Thus, upon completion of a multiple pass collection procedure, donated WB has been collected in the WB collection container, while biomarkers have been collected in the biomarker collection container.

In a first aspect, a blood pack donation system is configured for use with a LOC device for biomarker collection during WB donation including a blood collection container having a volume and at least one opening, a biomarker collection container having a volume and at least one opening, a first flow path having a first end connected to the opening in the blood collection container and a second end configured to be connected to a first outlet opening of the LOC device, a second flow path having a first end connected to the opening in the biomarker collection container and a second end configured to be connected to a second outlet opening of the LOC device, and a third flow path having a first end connected to a needle and a second end configured to be connected to an inlet opening of the LOC device. These components are present in first and second example systems, both of which are capable of being used in a single pass collection procedure.

In a second aspect, a blood pack donation system of the previous configuration may further include a fourth flow path having a first end connected to the first flow path at a location between the first end and second end of the first flow path and having a second end connected to the third flow path at a location between the first end and second end of the third flow path. The fourth flow path serves as the aforementioned bypass line or flow path between the donor and WB collection container, which permits bypassing the LOC device. The blood pack donation system also may include a plurality of flow control components that selectively control flow through the first flow path, third flow path and fourth flow path. The plurality of flow control components is selectively adjustable for use with a LOC device to provide a single pass collection procedure or a multiple pass collection procedure for collecting WB during a WB donation, while also collecting biomarkers. The multiple pass collection procedure includes a succession of adjustments of the plurality of flow control components.

It will be appreciated that a blood pack donation system configured for use with a LOC device for biomarker collection during WB donation may be constructed for use in advantageous, cost efficient and convenient single or multiple pass collection methods.

### BRIEF DESCRIPTION OF THE DRAWINGS

Figure 1 is a diagram of a first example blood pack donation system for use with a LOC device for biomarker collection during WB donation utilizing single pass collection.
Figure 2 is a diagram of a second example blood pack donation system for use with a LOC device for biomarker collection during WB donation utilizing single pass collection or multiple pass collection and illustrating flow control component settings for a single pass collection or a first step of a multiple pass collection.
Figure 3 is a diagram showing the system in Figure 2 and illustrating flow control component settings for a second step of a multiple pass collection.
Figure 4 is a diagram showing the system in Figure 2 and illustrating flow control component settings for a third step of a multiple pass collection.

### DETAILED DESCRIPTION

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

It will be appreciated that a blood pack donation system configured for use with a LOC device for biomarker collection during WB donation may be applicable for efficient and convenient use with a LOC device to screen for the presence of and collect biomarkers.

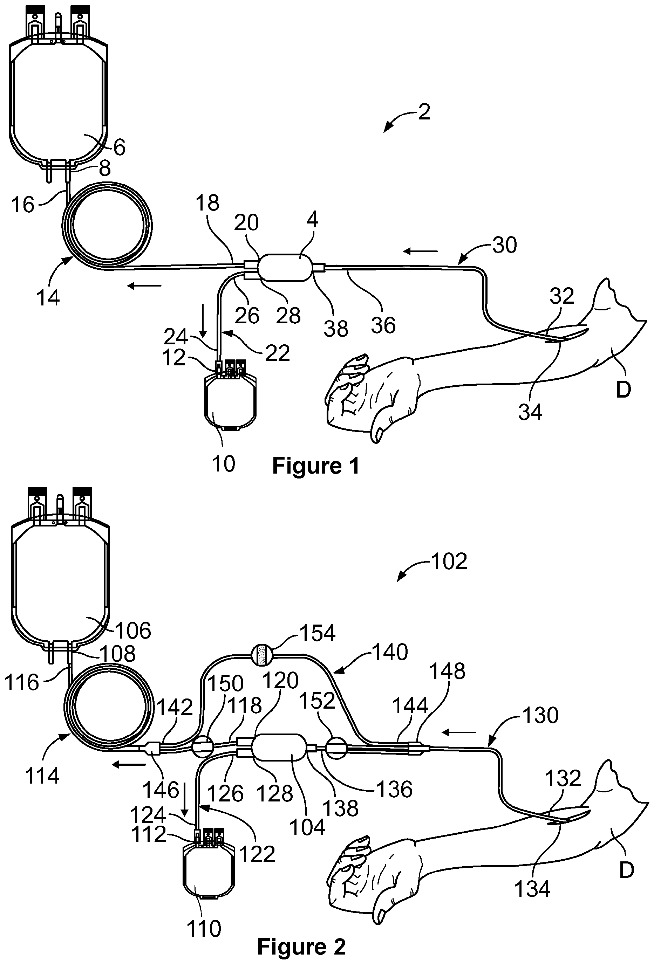

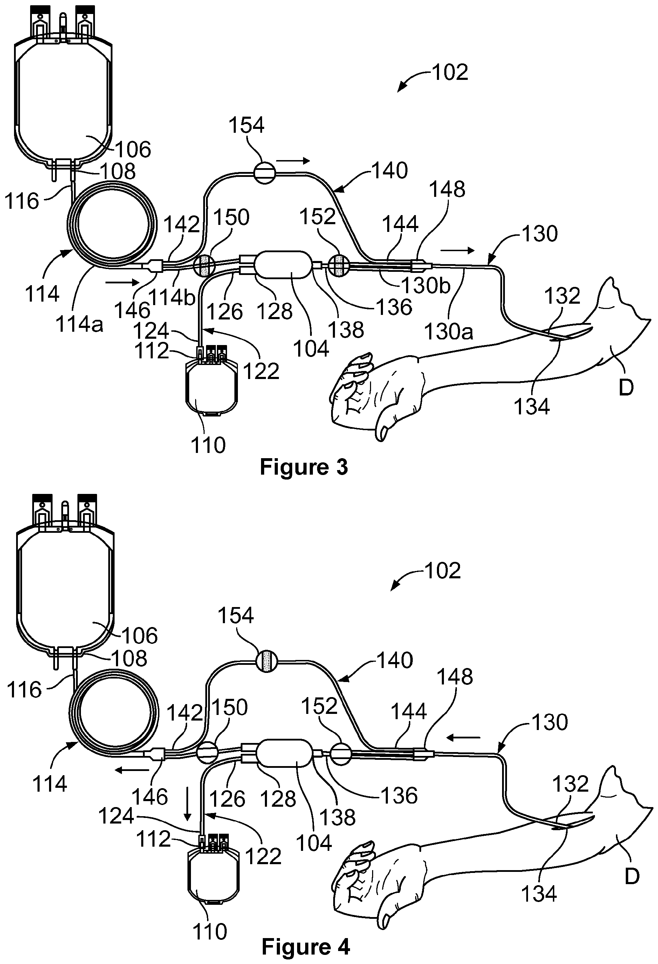

Figures 1-4 illustrate two example embodiments of such blood pack donation systems. The first embodiment, shown in Figure 1, provides for a single pass collection procedure via connection to a LOC device, which permits collecting WB during a WB donation, while screening for and collecting biomarkers. The second embodiment, shown in Figures 2-4, provides for a single pass collection procedure or an optional multiple pass collection procedure via connection to a LOC device. The second embodiment includes a plurality of flow control components wherein a single pass collection procedure comparable to that of the first embodiment may be performed with the plurality of flow control components having first selected settings. The second embodiment permits a multiple pass collection procedure that may be performed with the plurality of flow control components having a succession of further second and third selected settings, and which permits collecting WB during a WB donation, while screening for and collecting biomarkers.

Turning now to Figure 1, a blood pack donation system 2 is configured for use with a LOC device 4 for biomarker collection during WB donation. Thus, the blood pack donation system 2 preferably is presterilized and configured for use with any of a variety of LOC devices 4, which may be provided by various manufacturers for use in separating and screening for collection any of a number of different biomarkers. Thus, makers of LOC devices may construct or integrate their LOC devices for use with the system 2.

The system 2 includes a blood collection container 6 having a volume and at least one opening 8, and a biomarker collection container 10 having a volume and at least one opening 12. A first flow path 14 having a first end 16 is connected to the opening 8 in the blood collection container 6 and a second end 18 is configured to be connected to a first outlet opening 20 of the LOC device 4. A second flow path 22 having a first end 24 is connected to the opening 12 in the biomarker collection container 10 and a second end 26 configured to be connected to a second outlet opening 28 of the LOC device 4. A third flow path 30 having a first end 32 is connected to a needle 34 and a second end 36 configured to be connected to an inlet opening 38 of the LOC device 4. The needle 34 is configured for use with a donor D, for use in the manner of a routine WB donation procedure.

As noted above, the respective openings in the blood collection container 6 and biomarker collection container 10 may be preexisting or formed when connected to a respective flow path 16, 22. In addition, the flow paths may be constructed in the form of individual sections of tubing, or may be integrated into a cassette, whether in the form of channels or sections of tubing. It will be appreciated that the first example system 2 may have an alternative configuration in the form of a cassette, which may provide the respective flow paths already connected to a housing, which may be represented by the body 4, which in turn may accept a LOC device for connection to the respective flow paths for use.

As previously noted, the blood pack donation system 2 preferably is presterilized. The system 2 may be configured with integrated fixed connections between all or some of the components, such as via heat sealing, solvent bonding or other suitable means of connection. Connectors may be used to permanently join separated components. In a further alternative, all or some of the connections may be removably connected, such as by means of sterile unions using Luer-Lock connectors, or other suitable connection devices.

For example, the opening 8 of the blood collection container 6 may be removably connected to the first end 16 of the first flow path 14, and the first outlet opening 20 of the LOC device 4 may be removably connected to the second end 18 of the first flow path 14. Similarly, the opening 12 of the biomarker collection container 10 may be removably connected to the first end 24 of the second flow path 22, and the second outlet opening 28 of the LOC device 4 may be removably connected to the second end 26 of the second flow path 22. In turn, the needle 34 may be removably connected to the first end 32 of the third flow path 30, and the inlet opening 38 of the LOC device 4 may be removably connected to the second end 36 of the third flow path 30.

It will be appreciated that the system 2 may include fixed connections between components, but the system 2 may be configured for connection to the LOC device 4 at the point of use, so removable connections may be provided at least at the second end 18 of the first flow path 14, at the second end 26 of the second flow path 22 and at the second end 36 of the third flow path 30, for connection respectively to the LOC device 4 first outlet opening 20, second outlet opening 28 and inlet opening 38. Alternatively, manufacturers of LOC devices may fixedly connect such devices to the system 2, if desired.

Turning to Figures 2-4, a second example blood pack donation system 102 is configured for use with a LOC device 104 for biomarker collection during WB donation. The LOC device 104 may be similar to the LOC device 4 of the first example. The blood pack donation system 102 also preferably is presterilized and configured for use with any of a variety of LOC devices 104, from various manufacturers and for use in separating and screening for collection any of a number of different biomarkers. Accordingly, makers of LOC devices may construct or integrate their LOC devices for use with the system 102.

The system 102 includes a blood collection container 106 having a volume and at least one opening 108, and a biomarker collection container 110 having a volume and at least one opening 112. A first flow path 114 having a first end 116 is connected to the opening 108 in the blood collection container 106 and a second end 118 is configured to be connected to a first outlet opening 120 of the LOC device 104. A second flow path 122 having a first end 124 is connected to the opening 112 in the biomarker collection container 110 and a second end 126 configured to be connected to a second outlet opening 128 of the LOC device 104. A third flow path 130 having a first end 132 is connected to a needle 134 and a second end 136 configured to be connected to an inlet opening 138 of the LOC device 104. The needle 134 is configured for use with a donor D, for use in the manner of a routine WB donation procedure.

The second example system 102 further includes a fourth flow path 140 having a first end 142 connected to the first flow path 114 at a location between the first end 116 and second end 118 of the first flow path 114 and having a second end 144 connected to the third flow path 130 at a location between the first end 132 and second end 136 of the third flow path 130. The connections at the first end 142 and second end 144 of the fourth flow path 140 are shown in Figures 2-4 as Y-connectors 146 and 148, but are considered herein to be connections along lengths of the first flow path 114 and the third flow path 130. Consistent with the above discussion of the first example system 2, the flow path connections in the second example system 102 may be integrated or fixed, or removably connected, and the respective openings in the blood collection container 106 and biomarker collection container 110 may be preexisting or formed when connected to a respective flow path 116, 122. In addition, the flow paths may be constructed in the form of individual sections of tubing, or may be integrated into a cassette, whether in the form of channels or sections of tubing. As noted with respect to the first example system 2, a further alternative configuration of the second example system 102 may be in the form of a cassette, which may provide the respective flow paths already connected to a housing, which may be represented by the body 104, which in turn may accept a LOC device for connection to the respective flow paths for use.

Thus, similarly to the first example system 2, in the second system 102 the opening 108 of the blood collection container 106 may be removably connected to the first end 116 of the first flow path 114, and the first outlet opening 120 of the LOC device 104 may be removably connected to the second end 118 of the first flow path 114. Similarly, the opening 112 of the biomarker collection container 110 may be removably connected to the first end 124 of the second flow path 122, and the second outlet opening 128 of the LOC device 104 may be removably connected to the second end 126 of the second flow path 122. In turn, the needle 134 may be removably connected to the first end 132 of the third flow path 130, and the inlet opening 138 of the LOC device 104 may be removably connected to the second end 136 of the third flow path 130. The first end 142 of the fourth flow path 140 may be removably connected to the first flow path 114 and the second end 144 of the fourth flow path 140 may be removably connected to the third flow path 130.

As with the previous example, removable connections may be provided in the second example system 102 at least at the second end 118 of the first flow path 114, at the second end 126 of the second flow path 122 and at the second end 136 of the third flow path 130, for connection respectively to the first outlet opening 120, second outlet opening 128 and inlet opening 138 of the LOC device 104. Alternatively, manufacturers of LOC devices may fixedly connect such devices to the system 102, if desired.

The second example blood pack donation system 102 further includes a plurality of flow control components 150, 152 and 154 that selectively control flow through the first flow path 114, third flow path 130 and fourth flow path 140, respectively. The plurality of flow control components 150, 152 and 154 that selectively control flow through the first flow path 114, third flow path 130 and fourth flow path 140 is selectively adjustable for use with a LOC device 104 to provide a single pass collection procedure or a multiple pass collection procedure for collecting WB during a WB donation, while also collecting biomarkers.

The second example system 102 optionally is capable of use in a multiple pass collection procedure, which includes a succession of adjustments of the plurality of flow control components 150, 152 and 154. For example, as shown in Figure 2, the plurality of flow control components 150, 152 and 154 is adjusted in a first step to permit flow through the first flow path 114 to the blood collection container 106 and through the third flow path 130 to the LOC device 104, while preventing flow through the fourth flow path 140. With such settings of the flow control components, WB may flow through the third flow path 130, the LOC device 104, the first flow path 114 to collect WB in the WB collection container 106, while biomarkers flow through the second flow path 122 and are collected in the biomarker collection container 110. It will be appreciated that in step one, the flow control component 154 may partially or completely close the fourth flow path 140, so as to permit much or most of the WB to flow from the donor D to the blood collection container 106, while a lesser portion of the WB from the donor D must pass through the LOC device 104. This may permit a more rapid procedure, while still providing screening and collection of biomarkers from an adequate portion of the WB donation. Similarly, the flow control component 152 optionally may utilize a setting that selectively reduces or increases the flow through the LOC device 104, independently of the WB flow through the fourth flow path 140.

With the system 102, in a second step of a multiple pass collection procedure, the plurality of flow control components 150, 152 and 154 is further adjustable to permit reverse flow through a first portion 114a of the first flow path 114, through the fourth flow path 140 and through and a first portion 130a of the third flow path 130, while preventing reverse flow through a second portion 114b of the first flow path 114 and a second portion 130b of the third flow path 130. For example, this may be accomplished if the flow control component 154 permits the fourth flow path 140 to be partially or fully open, while the flow control components 150 and 152 fully close the second portion 114b of the first flow path 114 and the second portion 130b of the third flow path 130.

To complete the multiple pass collection procedure with the second example system 102, in a third step the plurality of flow control components 150, 152 and 154 is further adjustable and may be similar to the first step, so as to permit at least some flow through the first flow path 114 to the blood collection container 106 and through the third flow path 130 to the LOC device 104, while partially or fully preventing flow through the fourth flow path 140. Thus, WB may flow through the third flow path 130, the LOC device 104, the first flow path 114 to collect WB in the WB collection container 106, while biomarkers flow through the second flow path 122 and are collected in the biomarker collection container 110.

It will be appreciated that the plurality of flow control components 150, 152 and 154 further may include a plurality of respective clamps, such as Roberts-type clamps, pinch, slide or roller clamps, or other suitable types of clamps usable to selectively prevent or permit flow through a flow path that may be a section of tubing or a channel in a cassette or other suitable structure. Accordingly, the plurality of respective clamps 150, 152 and 154 is adjustable with respect to flow through the first flow path 114 to the blood collection container 106 and through the third flow path 130 to the LOC device 104, as well as the flow through the fourth flow path 140, for use in a single pass collection procedure or optionally in a multiple pass collection procedure.

Consistent with the above description, the system 102 may be further adjusted for a second step of a multiple pass collection procedure wherein the plurality of respective clamps 150, 152 and 154 is further adjustable to permit reverse flow from the blood collection container 106 through a first portion 114a of the first flow path 114, through the fourth flow path 140 and through a first portion 130a of the third flow path 130, while preventing reverse flow through a second portion 114b of the first flow path 114 and a second portion 130b of the third flow path 130.

It will be appreciate that in the second step of the multiple pass collection procedure, to prevent reverse flow through the LOC device 104, one of the respective clamps 152 is adjustable to selectively prevent reverse flow through the third flow path 130 at a location between the second end 136 of the third flow path 130 and the connection of the fourth flow path 140 to the third flow path 148. A further one of the respective clamps 150 is adjustable to selectively prevent reverse flow through the first flow path 114 at a location between the second end 118 of the first flow path 114 and the connection of the fourth flow path 140 to the first flow path 146.

Accordingly, example blood pack donation systems configured for use with a lab-on-a-chip device for biomarker collection during whole blood donation are disclosed and may be used to conduct a single pass collection procedure or a multiple pass collection procedure.

## Claims

1. A blood pack donation kit (2, 102) configured for use in whole blood donation and simultaneous use with a lab-on-a-chip device (4, 104) for separation and collection of biomarkers, wherein the kit comprises
a) a blood collection container (6, 106) having a volume and at least one opening (8, 108);
b) a biomarker collection container (10, 110) having a volume and at least one opening (12, 112);
c) a first flow path (14, 114) having a first end (16, 116) connected to the opening (8, 108) in the blood collection container (6, 106) and a second end (18, 118) configured to be connected to a first outlet opening (20, 120) of a lab-on-a-chip device (4, 104);
d) a second flow path (22, 122) having a first end (24, 124) connected to the opening (12, 112) in the biomarker collection container (10, 110) and a second end (26, 126) configured to be connected to a second outlet opening (28, 128) of the lab-on-a-chip device (4, 104); and
e) a third flow path (30, 130) having a first end (32, 132) connected to a needle (34, 134) and a second end (36, 136) configured to be connected to an inlet opening (38, 138) of the lab-on-a-chip device (4, 104).

2. The blood pack donation kit (2, 102) of claim 1 wherein the opening (8, 108) of the blood collection container (6, 106) is removably connected to the first end (16, 116) of the first flow path (14, 114).

3. The blood pack donation kit (2, 102) of claim 1 or 2 wherein the first outlet opening (20, 120) of the lab-on-a-chip device (4, 104) is removably connected to the second end (18, 118) of the first flow path (14, 114).

4. The blood pack donation kit (2, 102) of any of claims 1-3 wherein the opening (12, 112) of the biomarker collection container (10, 110) is removably connected to the first end (24, 124) of the second flow path (22, 122).

5. The blood pack donation kit (2, 102) of any of claims 1-4 wherein the second outlet opening (28, 128) of the lab-on-a-chip device (4, 104) is removably connected to the second end (26, 126) of the second flow path (22, 122).

6. The blood pack donation kit (2, 102) of any of claims 1-5 wherein the needle (34, 134) is removably connected to the first end (32, 132) of the third flow path (30, 130).

7. The blood pack donation kit (2, 102) of any of claims 1-6 wherein the inlet opening (38, 138) of the lab-on-a-chip device (4, 104) is removably connected to the second end (36, 136) of the third flow path (30, 130).

8. The blood pack donation kit (102) of any of claims 1-7 further comprising a fourth flow path (140) having a first end (142) connected to the first flow path (114) at a location (146) between the first end (116) and second end (118) of the first flow path (114) and having a second end (144) connected to the third flow path (130) at a location (148) between the first end (132) and second end (136) of the third flow path (130).

9. The blood pack donation kit (102) of claim 8 wherein the first end (142) of the fourth flow path (140) is removably connected to the first flow path (114) and the second end (144) of the fourth flow path (114) is removably connected to the third flow path (130).

10. The blood pack donation kit (102) of claim 9 further comprising a plurality of flow control components (150, 152, 154) that selectively control flow through the first flow path (114), third flow path (130) and fourth flow path (140).

11. The blood pack donation kit (102) of claim 10 wherein the plurality of flow control components (150, 152, 154) that selectively control flow through the first flow path (114), third flow path (130) and fourth flow path (140) is selectively adjustable for use with a lab-on-a-chip device (104) to provide a single pass collection procedure or a multiple pass collection procedure for collecting whole blood during a whole blood donation, while also collecting biomarkers.

12. The blood pack donation kit (102) of claim 11 wherein the multiple pass collection procedure includes a succession of adjustments of the plurality of flow control components (150, 152, 154).

13. The blood pack donation kit (102) of claim 11 wherein the plurality of flow control components (150, 152, 154) is adjustable to permit flow through the first flow path to the blood collection container and through the third flow path to the lab-on-a-chip device, while permitting partial flow or preventing flow through the fourth flow path.

14. The blood pack donation kit (102) of claim 13 wherein the plurality of flow control components (150, 152, 154) is further adjustable to permit reverse flow through a first portion of the first flow path (114) between the first end (116) of the first flow path (114) and the location (146), through the fourth flow path (140) and through and a first portion of the third flow path (130) between the first end (132) of the third flow path (130) and the location (148), while preventing reverse flow through a second portion of the first flow path (114) between the second end (118) of the first flow path (114) and the location (146), and a second portion of the third flow path (130) between the second end (136) of the third flow path (130) and the location (148).

15. The blood pack donation kit (102) of claim 10 wherein the plurality of flow control components (150, 152, 154) further comprises a plurality of respective clamps.

## Patentansprüche

1. Blutpackungsspende-Kit (2, 102) zur Verwendung bei der Vollblutspende und zur gleichzeitigen Verwendung mit einer Lab-on-a-Chip-Vorrichtung (4, 104) zur Trennung und Sammlung von Biomarkern, wobei das Kit umfasst:
a) einen Blutsammelbehälter (6, 106) mit einem Volumen und zumindest einer Öffnung (8, 108);
b) einen Biomarker-Sammelbehälter (10, 110) mit einem Volumen und zumindest einer Öffnung (12, 112);
c) einen ersten Strömungspfad (14, 114) mit einem ersten Ende (16, 116), das mit der Öffnung (8, 108) in dem Blutsammelbehälter (6, 106) verbunden ist, und mit einem zweiten Ende (18, 118) zur Verbindung mit einer ersten Auslassöffnung (20, 120) einer Lab-on-a-Chip-Vorrichtung (4, 104);
d) einen zweiten Strömungspfad (22, 122) mit einem ersten Ende (24, 124), das mit der Öffnung (12, 112) in dem Biomarker-Sammelbehälter (10, 110) verbunden ist, und mit einem zweiten Ende (26, 126) zur Verbindung mit einer zweiten Auslassöffnung (28, 128) der Lab-on-a-Chip-Vorrichtung (4, 104); und
e) einen dritten Strömungspfad (30, 130) mit einem ersten Ende (32, 132), das mit einer Nadel (34, 134) verbunden ist, und mit einem zweiten Ende (36, 136) zur Verbindung mit einer Einlassöffnung (38, 138) der Lab-on-a-Chip-Vorrichtung (4, 104).

2. Blutpackungsspende-Kit (2, 102) nach Anspruch 1, wobei die Öffnung (8, 108) des Blutsammelbehälters (6, 106) lösbar mit dem ersten Ende (16, 116) des ersten Strömungspfads (14, 114) verbunden ist.

3. Blutpackungsspende-Kit (2, 102) nach Anspruch 1 oder 2, wobei die erste Auslassöffnung (20, 120) der Lab-on-a-Chip-Vorrichtung (4, 104) lösbar mit dem zweiten Ende (18, 118) des ersten Strömungspfads (14, 114) verbunden ist.

4. Blutpackungsspende-Kit (2, 102) nach einem der Ansprüche 1 bis 3, wobei die Öffnung (12, 112) des Biomarker-Sammelbehälters (10, 110) lösbar mit dem ersten Ende (24, 124) des zweiten Strömungspfades (22, 122) verbunden ist.

5. Blutpackungsspende-Kit (2, 102) nach einem der Ansprüche 1 bis 4, wobei die zweite Auslassöffnung (28, 128) der Lab-on-a-Chip-Vorrichtung (4, 104) lösbar mit dem zweiten Ende (26, 126) des zweiten Strömungspfades (22, 122) verbunden ist.

6. Blutpackungsspende-Kit (2, 102) nach einem der Ansprüche 1 bis 5, wobei die Nadel (34, 134) lösbar mit dem ersten Ende (32, 132) des dritten Strömungspfades (30, 130) verbunden ist.

7. Blutpackungsspende-Kit (2, 102) nach einem der Ansprüche 1 bis 6, wobei die Einlassöffnung (38, 138) der Lab-on-a-Chip-Vorrichtung (4, 104) lösbar mit dem zweiten Ende (36, 136) des dritten Strömungspfades (30, 130) verbunden ist.

8. Blutpackungsspende-Kit (2, 102) nach einem der Ansprüche 1 bis 7, ferner umfassend einen vierten Strömungspfad (140) mit einem ersten Ende (142), das an einer Stelle (146) zwischen dem ersten Ende (116) und dem zweiten Ende (118) des ersten Strömungspfads (114) mit dem ersten Strömungspfad (114) verbunden ist, und mit einem zweiten Ende (144), das an einer Stelle (148) zwischen dem ersten Ende (132) und dem zweiten Ende (136) des dritten Strömungspfads (130) mit dem dritten Strömungspfad (130) verbunden ist.

9. Blutpackungsspende-Kit (2, 102) nach Anspruch 8, wobei das erste Ende (142) des vierten Strömungspfades (140) lösbar mit dem ersten Strömungspfad (114) verbunden ist und das zweite Ende (144) des vierten Strömungspfades (114) lösbar mit dem dritten Strömungspfad (130) verbunden ist.

10. Blutpackungsspende-Kit (2, 102) nach Anspruch 9, ferner umfassend mehrere Strömungssteuerungskomponenten (150, 152, 154), die die Strömung durch den ersten Strömungspfad (114), den dritten Strömungspfad (130) und den vierten Strömungspfad (140) selektiv steuern.

11. Blutpackungsspende-Kit (102) nach Anspruch 10, wobei die mehreren Strömungssteuerungskomponenten (150, 152, 154), die die Strömung durch den ersten Strömungspfad (114), den dritten Strömungspfad (130) und den vierten Strömungspfad (140) selektiv steuern, zur Verwendung mit einer Lab-on-a-Chip-Vorrichtung (104) selektiv einstellbar sind, um ein Ein-Durchgang-Sammelverfahren oder ein Mehr-Durchgang-Sammelverfahren zum Sammeln von Vollblut während einer Vollblutspende bereitzustellen, währenddem auch Biomarker gesammelt werden.

12. Blutpackungsspende-Kit (102) nach Anspruch 11, wobei das Mehr-Durchgang-Sammelverfahren eine Abfolge von Einstellungen der mehreren Strömungssteuerungskomponenten (150, 152, 154) umfasst.

13. Blutpackungsspende-Kit (102) nach Anspruch 11, wobei die mehreren Strömungssteuerungskomponenten (150, 152, 154) so einstellbar sind, dass sie eine Strömung durch den ersten Strömungspfad zum Blutsammelbehälter und durch den dritten Strömungspfad zur Lab-on-a-Chip-Vorrichtung ermöglichen und dabei eine Teilströmung durch den vierten Strömungspfad zulassen oder eine Strömung durch diesen verhindern.

14. Blutpackungsspende-Kit (102) nach Anspruch 13, wobei die mehreren Strömungssteuerungskomponenten (150, 152, 154) ferner so einstellbar sind, dass sie eine Rückströmung durch einen ersten Abschnitt des ersten Strömungspfads (114) zwischen dem ersten Ende (116) des ersten Strömungspfads (114) und der Stelle (146), durch den vierten Strömungspfad (140) und durch einen ersten Abschnitt des dritten Strömungspfads (130) zwischen dem ersten Ende (132) des dritten Strömungspfads (130) und der Stelle (148) zulassen, während sie eine Rückströmung durch einen zweiten Abschnitt des ersten Strömungspfads (114) zwischen dem zweiten Ende (118) des ersten Strömungspfads (114) und der Stelle (146) und durch einen zweiten Abschnitt des dritten Strömungspfads (130) zwischen dem zweiten Ende (136) des dritten Strömungspfads (130) und der Stelle (148) verhindern.

15. Blutpackungsspende-Kit (102) nach Anspruch 10, wobei die mehreren Strömungssteuerungskomponenten (150, 152, 154) ferner mehrere jeweilige Klemmen umfassen.

## Revendications

1. Ensemble de don de poches de sang (2, 102) configuré pour être utilisé lors du don de sang total et pour être utilisé simultanément avec un dispositif de laboratoire sur puce (4, 104) pour la séparation et la collecte de biomarqueurs, dans lequel l'ensemble comprend
a) un récipient de collecte de sang (6, 106) présentant un volume et au moins une ouverture (8, 108) ;
b) un récipient de collecte de biomarqueurs (10, 110) présentant un volume et au moins une ouverture (12, 112) ;
c) un premier trajet d'écoulement (14, 114) présentant une première extrémité (16, 116) reliée à l'ouverture (8, 108) dans le récipient de collecte de sang (6, 106) et une seconde extrémité (18, 118) configurée pour être reliée à une première ouverture de sortie (20, 120) d'un dispositif laboratoire sur puce (4, 104) ;
d) un deuxième trajet d'écoulement (22, 122) présentant une première extrémité (24, 124) reliée à l'ouverture (12, 112) dans le récipient de collecte de biomarqueurs (10, 110) et une seconde extrémité (26, 126) configurée pour être reliée à une deuxième ouverture de sortie (28, 128) du dispositif laboratoire sur puce (4, 104) ; et
e) un troisième trajet d'écoulement (30, 130) présentant une première extrémité (32, 132) reliée à une aiguille (34, 134) et une seconde extrémité (36, 136) configurée pour être reliée à une ouverture d'entrée (38, 138) du dispositif laboratoire sur puce (4, 104).

2. Ensemble de don de poches de sang (2, 102) selon la revendication 1, dans lequel l'ouverture (8, 108) du récipient de collecte de sang (6, 106) est reliée de manière amovible à la première extrémité (16, 116) du premier trajet d'écoulement (14, 114).

3. Ensemble de don de poches de sang (2, 102) selon la revendication 1 ou 2, dans lequel la première ouverture de sortie (20, 120) du dispositif de laboratoire sur puce (4, 104) est reliée de manière amovible à la seconde extrémité (18, 118) du premier trajet d'écoulement (14, 114).

4. Ensemble de don de poches de sang (2, 102) selon l'une quelconque des revendications 1-3, dans lequel l'ouverture (12, 112) du récipient de collecte de biomarqueurs (10, 110) est reliée de manière amovible à la première extrémité (24, 124) du deuxième trajet d'écoulement (22, 122).

5. Ensemble de don de poches de sang (2, 102) selon l'une quelconque des revendications 1-4, dans lequel la seconde ouverture de sortie (28, 128) du dispositif de laboratoire sur puce (4, 104) est reliée de manière amovible à la seconde extrémité (26, 126) du deuxième trajet d'écoulement (22, 122).

6. Ensemble de don de poches de sang (2, 102) selon l'une quelconque des revendications 1-5, dans lequel l'aiguille (34, 134) est reliée de manière amovible à la première extrémité (32, 132) du troisième trajet d'écoulement (30, 130).

7. Ensemble de don de poches de sang (2, 102) selon l'une quelconque des revendications 1-6, dans lequel l'ouverture d'entrée (38, 138) du dispositif de laboratoire sur puce (4, 104) est reliée de manière amovible à la seconde extrémité (36, 136) du troisième trajet d'écoulement (30, 130).

8. Ensemble de don de poches de sang (102) selon l'une quelconque des revendications 1-7, comprenant en outre un quatrième trajet d'écoulement (140) présentant une première extrémité (142) reliée au premier trajet d'écoulement (114) sur un emplacement (146) entre la première extrémité (116) et la seconde extrémité (118) du premier trajet d'écoulement (114) et présentant une seconde extrémité (144) reliée au troisième trajet d'écoulement (130) sur un emplacement (148) entre la première extrémité (132) et la seconde extrémité (136) du troisième trajet d'écoulement (130).

9. Ensemble de don de poches de sang (102) selon la revendication 8, dans lequel la première extrémité (142) du quatrième trajet d'écoulement (140) est reliée de manière amovible au premier trajet d'écoulement (114) et la seconde extrémité (144) du quatrième trajet d'écoulement (114) est reliée de manière amovible au troisième trajet d'écoulement (130).

10. Ensemble de don de poches de sang (102) selon la revendication 9, comprenant en outre une pluralité de composants de commande d'écoulement (150, 152, 154), qui commandent au choix l'écoulement à travers le premier trajet d'écoulement (114), le troisième trajet d'écoulement (130) et le quatrième trajet d'écoulement (140).

11. Ensemble de don de poches de sang (102) selon la revendication 10, dans lequel la pluralité de composants de commande d'écoulement (150, 152, 154) qui commandent au choix l'écoulement à travers le premier trajet d'écoulement (114), le troisième trajet d'écoulement (130) et le quatrième trajet d'écoulement (140) peut être ajustée au choix pour une utilisation avec un dispositif laboratoire sur puce (104) pour fournir une procédure de collecte à passage unique ou une procédure de collecte à passages multiples pour collecter du sang total pendant un don de sang total, tout en collectant également des biomarqueurs.

12. Ensemble de don de poches de sang (102) selon la revendication 11, dans lequel la procédure de collecte à passages multiples comporte une succession d'ajustements de la pluralité de composants de commande d'écoulement (150, 152, 154).

13. Ensemble de don de poches de sang (102) selon la revendication 11, dans lequel la pluralité de composants de commande d'écoulement (150, 152, 154) peut être ajustée pour permettre un écoulement à travers le premier trajet d'écoulement au récipient de collecte de sang et à travers le troisième trajet d'écoulement au dispositif laboratoire sur puce, tout en permettant un écoulement partiel ou en empêchant un écoulement à travers le quatrième trajet d'écoulement.

14. Ensemble de don de poches de sang (102) selon la revendication 13, dans lequel la pluralité de composants de commande d'écoulement (150, 152, 154) peut être en outre ajustée pour permettre un écoulement inverse à travers une première partie du premier trajet d'écoulement (114) entre la première extrémité (116) du premier trajet d'écoulement (114) et l'emplacement (146), à travers le quatrième trajet d'écoulement (140) et à travers et une première partie du troisième trajet d'écoulement (130) entre la première extrémité (132) du troisième trajet d'écoulement (130) et l'emplacement (148), tout en empêchant un écoulement inverse à travers une deuxième partie du premier trajet d'écoulement (114) entre la seconde extrémité (118) du premier trajet d'écoulement (114) et l'emplacement (146), et une deuxième partie du troisième trajet d'écoulement (130) entre la seconde extrémité (136) du troisième trajet d'écoulement (130) et l'emplacement (148).

15. Ensemble de don de poches de sang (102) selon la revendication 10, dans lequel la pluralité de composants de commande d'écoulement (150, 152, 154) comprend en outre une pluralité de pinces respectives.
